(19) **Europäisches Patentamt**

**European Patent Office**

**Office européen des brevets**

(11) **EP 0 670 165 B1**

(12) **EUROPEAN PATENT SPECIFICATION**

(45) Date of publication and mention
of the grant of the patent:
**12.12.2001 Bulletin 2001/50**

(51) Int Cl.[7]: **A61K 38/46**

(21) Application number: **95830040.2**

(22) Date of filing: **15.02.1995**

(54) **Use of seminal ribonuclease as antimetastatic compound**

Verwendung der Seminalribonuklease als antimetastatische Verbindung

Utilisation de ribonucléase séminale comme composé antimétastatique

(84) Designated Contracting States:
**AT BE CH DE DK ES FR GB GR IE LI LU MC NL PT SE**

(30) Priority: **16.02.1994 IT RM940072**

(43) Date of publication of application:
**06.09.1995 Bulletin 1995/36**

(73) Proprietor: **UNIVERSITA' DEGLI STUDI DI NAPOLI I-80138 Napoli (IT)**

(72) Inventors:
• **Vecchio, Giancarlo, Universita di Napoli I-80131 Napoli (IT)**
• **Lacetti, Paolo, Universita di Napoli I-80131 Napoli (IT)**
• **D'Alessio, Guiseppe, Universita di Napoli I-80134 Napoli (IT)**

(74) Representative: **de Simone, Domenico et al Ing. Barzanò & Zanardo Roma S.p.A. Via Piemonte 26 00187 Roma (IT)**

(56) References cited:
**DE-A- 2 911 867**

• **CANCER RESEARCH, vol. 52, 1 September 1992, pages 4582-4586, XP002000753 P. LACCETTI ET AL.: "IN VIVO AND IN VITRO GROWTH-INHIBITORY EFFECT OF BOVINE SEMINAL RIBONUCLEASE ON A SYSTEM OF RAT THYROID EPITHELIAL TRANSFORMED CELLS AND TUMORS"**
• **TIBS, vol. 16, March 1991, pages 104-106, XP002000754 G. D'ALESSIO ET AL.: "SEMINAL RNASE: A UNIQUE MEMBER OF THE RIBONUCLEASE SUPERFAMILY"**
• **CANCER RESEARCH, vol. 54, 15 August 1994, pages 4253-4256, XP002000755 P. LACCETTI ET AL.: "SEMINAL RIBONUCLEASE INHIBITS TUMOR GROWTH AND REDUCES THE METASTATIC POTENTIAL OF LEWIS LUNG CARCINOMA"**

**Description**

This invention relates to the use of seminal ribonuclease to develop pharmaceutic compositions to be used in the antimetastatic therapy of tumors.

It is well known from the previous art that some ribonucleases (RNase) exert an antitumoral activity, such as RNase from <u>Rana pipiens</u> eggs, defined as "onconase" (1-3).

Seminal RNase (BS-RNase) (4) shows a cytotoxic activity against tumoral cells <u>in vitro</u> (5-7). However, till now this effect could not be confirmed also <u>in vivo,</u> thus preventing the BS-RNase to be used as antimetastatic compound, as well as to define the BS-RNase amount as effective, but not dangerous.

The authors assayed the BS-RNase activity against various tumors <u>in vivo</u> and found a significant and specific effect on metastasis formations. Such experiments, using the Lewis's lung carcinoma cell line (8) as metastasis inductor, show that a BS-RNase systematic administration lowers considerably lung metastasis occurence.

Accordingly, it is an object of the invention the use of BS-RNase to make pharmaceutic compositions for the therapy of tumor metastasis. The BS-RNase term, as used here, means to comprise any natural, synthesized or from recombinant DNA seminal ribonuclease, obtained using well known techniques by the skilled in the art.

BS-RNase is comprised in said compositions in pharmaceutically acceptable amounts, preferably ranging from 5 to 24 µg/g of body weight of the subject, more preferably in a range from 10 to 20 µg/g.

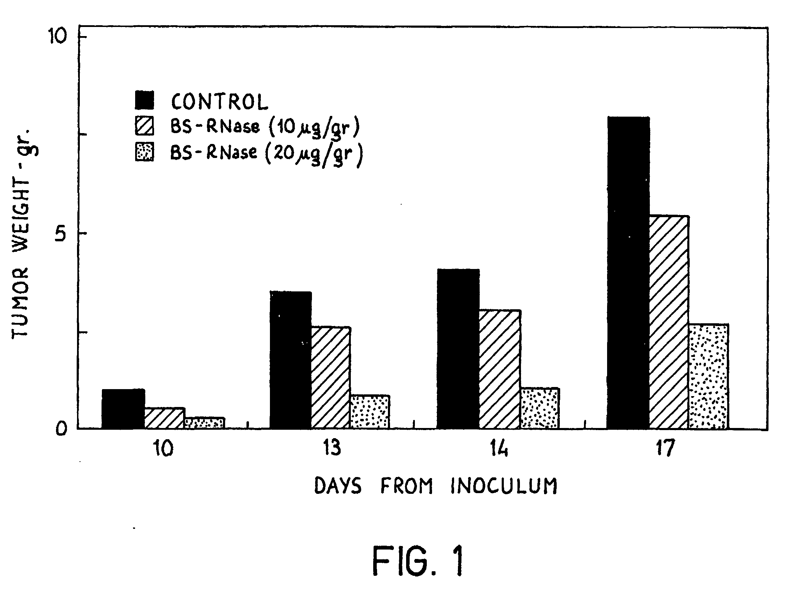

The invention will now be described exemplarily, but not limitately, by reference to the fig.1, wherein a bar graph of the volume growth of the 3LL tumors in treated rats with two BS-RNase different concentrations is showed.

MATERIALS

Bovine seminal RNase was purified from bovine seminal vesicles, according to the methods, as in Tamburrini and al. (9). The purity level of each material was tested through an electrophoresis procedure on polyacrilamide gel in SDS (sodium dodecylsulfate), as well as through a HPLC procedure (9) on S 5/5 single column (Pharmacia) procedure. Rnase A (type IIIA) was supplied by Sigma Chemical Co.

The 3LL subline, that was derived <u>in vivo</u> from Lewis's lung carcinomas, was injected in one month aged, C57BL/6 NCr1BR male mice. Each mouse was administered by 0.25 ml of a suspension, comprising $2.5 \times 10^5$ living tumor cells, through an injection in the leg muscle. A standard mechanical procedure was used to obtain a single cell suspension from solid tumors, as described in (12). Also a cell line, derived from the <u>in vivo</u> 3LL line was used. The 3LL cell line was cultivated as described in (8).

METASTASIS INHIBITION EFFECT OF SEMINAL RNase <u>IN VIVO</u>

Seminal RNase activity to inhibit <u>in vivo</u> the neoplastic growth was tested on C57BL/6NCr1BR male mice, in which lung carcinomas were induced. The Lewis's lung carcinoma was induced through injections of a unique 3LL cell suspension in a one month aged C57BL/6NCr1BR male mice. The tumor was induced through injecting daily 0.025 ml of a suspension, comprising $2.5 \times 10^5$ living 3LL cells in a PBS sterile solution. On the first day, RNase was administered in a PBS sterile solution at two different concentrations, and namely 10 and 20 µg/g of the mouse body weight, to 10 mice per group. Rnase A, a RNase having no antitumoral activity, or a PBS solution, was administered as control (to 10 mice per group). The injections, as above, were repeated at 72 hour intervals. On the seventieth day, when the tumors were appr. one third of the mouse body weight, mice were sacrified and the tumors and the lungs picked up, weighed and fixed in formaldehyde, 10%, and in Bouin solution, as described in (11). Blood samples also were picked up in order to obtain the main hematologic parameters.

During the treatment, the tumor volume was calculated, using the following formula (12):

$$V \text{ (volume)} = \text{thickness}^2 \times 0.5 \text{ (length)}.$$

The average standard error to define the tumor weight and the lung metastasis amount was calculated as follows:

$$SE = S/\sqrt{n}$$

wherein S is the standard error and n is the number of mice.

As shown in table 1 and in fig.1, the seminal RNase antitumoral effect is dose dependent; the tumor growth inhibition was appr. 31% when 10 µg of seminal RNase per gram of body weight were administered, while the tumor growth inhibitions amounted to appr. 66%, when 20 µg of seminal RNase per gram of body weight were administered.

Table 1

| A | Tumor weight(g) | Inhibition | No. of metastasis | Inhibition |
|---|---|---|---|---|
| Control | 8.0±0.11 | - | 24 ±0.95 | - |
| BS-RNase* | 5.5±0.17 | 31.2% | 8 ±0.39 | 66.7% |
| BS-RNase** | 2.7±0.14 | 66.2% | 2 ±0.18 | 91.7% |
| B | Tumor weight(g) | Inhibition | No. of metastasis | Inhibition |
| Control | 8.52±0.10 | - | 27 ±1.40 | - |
| BS-RNase** | 6.07±0.20 | 28.8% | 13 ±0.78 | 51.8% |

* 10 µg per gram of body weight

** 20 µg per gram of body weight

A significant effect of seminal RNase was observed in respect of lung metastasis induced by 3LL cell intramuscular injections. As shown in Table 1A, a seminal RNase amount of 10 µg per gram of body weight inhibited appr. the 67% of the lung metastases to occur, while an amount of 20 µg per gram of body weight inhibited more then 90% of metastases to occur. Control consisted of not treated mice, or treated mice with a PBS solution. Furthermore lung metastases, as detected in enzyme treated mice, had smaller sizes than the sizes of metastases of not treated animals.

The antimetastatic action of seminal RNase was also investigated by delaying the enzyme treatment up to five days after the tumoral cell inoculum, when the tumor weighed already appr. one gram. As shown in Table 1B, a significant effect was detected, i.e., a 52% reduction of the number of metastases.

In order to investigate whether seminal RNase treatment had a toxic effect on treated animals, blood samples were taken from each mouse, and tested for main hematologic parameters. Anemia and leukocytosis were detected in not treated mouse blood samples, while such alterations lowered in mice treated with 10 µg per gram of body weight, and were fully reversed in mice treated with the max. amount of 20 µg per gram of body weight. Moreover, neither changements in the mouse physic aspect, nor in its behavior was observed, except a small body weight lowering. The enzyme administration to healthy mice led no alterations to the hematologic parameters.

Therefore, seminal RNase is a powerful antimetastasis agent. Furthermore, seminal RNase is effective against the tumor after a systematic administration, a tumor consistent implantation and a tumor size significant increase. This confirms that the enzyme action is not due simply to a topic cytostatic effect.

REFERENCES

1. Ardelt, W., Mikulski, S.M. and Shogen, K.: "J. Biol. Chem." 266, 245-251 (1991)

2.Mikulski, S.M, Ardelt, W., Shogen, K., Bernstein, E.H. and Mendluke, H.: "J. Natl. Cancer Inst." 82, 151-153, (1990)

3. Mikulski, S.M., Viara, A., Darzynkiewicz, Z. and Shogen, K.: "Br. J. Cancer" 66, 304-310 (1992)

4.D'Alessio, G., Di Donato, A., Parente, A. and Piccoli, R.: "Trends Biochem. Sci." 16, 104-106 (1991)

5.Matousek, J.: "Experientia (Basel) 29, 858-859 (1973)

6.Vescia, S., Tramontano, D., Augusti-Tocco, G. and D'Alessio, G.: "Cancer Res." 40, 3740-3744 (1980)

7.Laccetti, P., Portella, G., Mastronicola, M.R., Russo, A., Piccoli, R., D'Alessio, G. and Vecchio, G.: "Cancer Research" 52, 4582-4586 (1992)

8.Zupi, G., Mauro, F. and Sacchi, A. "Br. J. Cancer" 41, 309-310 (1980)

9.Tamburrini, M., Piccoli, R., De Prisco, R., Di Donato, A. and D'Alessio, G.: "Ital. J. Biochem." 32, 22-32 (1985)

10.Portella, G., Ferulano, G., Santoro, M., Grieco, M., Fusco, A. and Vecchio, G.: "Oncogene" 4, 181-188 (1989)

11. Ambesi-Impiombato, F.S, Parks,d L.A.M. and Coon H.G.: "Procl. Natl. Acad. Sci. USA" 77, 3455-3459 (1980)

12.Corsi, A., Calabresi, F. and Greco, C.. "Br. J. Cancer" 38, 631-633 (1978)

13.Sacchi, A., Calabresi, F., Greco, C. and Zupi G.:"Invasion Metastasis" 1, 227-238 (1981)

14.Inase T, Tamura, M., Fujita, K., Kodame, S. and Tanaka, S.:"Cancer Res." 53, 2566-2570 (1993)

**Claims**

**1.** Use of seminal ribonuclease (BS-RNase) in pharmaceutically acceptable amounts for the manufacture of pharmaceutical compositions for the therapy of tumor metastases.

2. Use of seminal ribonuclease (BS-RNase) according to claim 1, wherein said BS-RNase is in a dosage ranging from 5 to 25 µg per gram of body weight.

3. Use of seminal ribonuclease (BS-RNase) according to claim 2, wherein said BS-RNase is in a dosage ranging from 10 to 20 µg per gram of body weight.

**Patentansprüche**

1. Verwendung von Seminalribonuklease (BS - Rnase) in einer pharmazeutisch verträglichen Menge für die Vorbereintung von pharmazeutischen Verbindungen für die Therapie von Tumormetastasen.

2. Verwendung von Seminalribonuklease (BS - Rnase) nach Anspruch 1, worin die vorgenannte BS - Rnase in einer Dosiermenge von 5 bis 25 µg/g - Körpergewicht, verabreicht wird.

3. Verwendung von Seminalribonuklease (BS - Rnase) nach Anspruch 2, worin die vorgenannte BS - Rnase in einer Dosiermenge von 10 bis 20 µg/g - Körpergewicht, verabreicht wird.

**Revendications**

1. Utilisation de ribonucléase séminale (BS - RNase) dans une quantité pharmaceutiquement tolérable pour la préparation de compositions pharmaceutiques pour la thérapie des métastases tumorales.

2. Utilisation de ribonucléase séminale (BS - RNase) selon la revendication 1, dans laquelle ladite BS - RNase est dans un dosage entre 5 et 25 µg/g de poids corporel.

3. Utilisation de ribonucléase séminale (BS - RNase) selon la revendication 2, dans laquelle ladite BS - RNase est dans un dosage entre 10 et 20 µg/g de poids corporel.

FIG. 1

EP 0 670 165 B1